# EUROPEAN PATENT APPLICATION

(11) **EP 1 875 923 A2**
(43) Date of publication of application: **09.01.2008**
(21) Application number: 07111646.1
(22) Date of filing: 03.07.2007
(51) Int. Cl.: A61K 38/17, A61P 25/00

(54) **Use of fractalkine for the production of a neuroprotector drug against cell death**

(30) Priority: 06.07.2006 IT RM20060353
(71) Applicant: Istituto Neurologico Mediterraneo NEUROMED S.r.l., 86077 Pozzilli IS (IT); Universita'Degli Studi di Roma "La Sapienza", 00185 Roma (IT)
(72) Inventor: Battaglia, Giuseppe, 95040 Mazzarrone CT (IT); Biagioni, Francesca, 57027 S. Vincenzo (LI) (IT); Busceti, Carla, Letizia, 86079 Venafro IS (IT); Cantore, Giampaolo, 00162 Roma (IT); Eusebi, Fabrizio, 00198 Roma (IT); Lauro, Clotilde, 00040 Ariccia RM (IT); Fornai, Francesco, 56030 Morrona PI (IT); Limatola, Cristina, 00040 Marino RM (IT); Nicoletti, Ferdinando, 95021 Aci Castello CT (IT); Catalano, Myriam, 00142 Rome (IT)
(74) Representative: Germinario, Claudio

(57) **Abstract**

The present invention relates to the use of an endogenous protein of immunitary origin, fractalkine, as a neuroprotective agent against glutamate toxicity in case of acute cerebral ischemia and spinal cord lesions. The agent is useful also in neurosurgery during operations on the brain as neuroprotective agent in case of blockage of blood circulation in the localized brain region subject to surgical intervention. Neuroprotection is exerted in the interval of a few hours after the ischemic insult and thus should be considered unique in the described pathophysiological applications.

## Description

### State of the art

The present invention has been developed performing scientific research to investigate the role of some chemokines and of their receptors which are expressed in the central nervous system (CNS) under pathophysiological conditions. Chemokines play many functions on the brain: they regulate both the neuronal migration and the synaptic neurotransmission, and they exert a neuro protective and trophic action. The main goal of this project in which this invention has been developed was to discover the molecular mechanisms which represent the basis of the functional effects mediated by chemokines on the synaptic transmission and on the development of the nervous system (NS). The research project derives from investigations on the chemokine known as fractalkine (F) and its receptor (CX₃CR₁ or CX₃CR₁) both expressed in CNS where they regulate neurotransmission. Fractalkine contributes to cell communication, mediates signalling pathways between neurons or between neurons and glial cells and participates to mechanisms of neurogenesis and cell differentiation during the NS development. Furthermore fractalkine plays an important role on the pathogenesis of many neuroinflammatory diseases. Inventors of the present invention worked on the glutamate-induced excitotoxicity on hippocampal neurons in culture *(in vitro* conditions), and demonstrated in a recent paper (Limatola et al., J. Neuroimmunology, 2005) that fractalkine increases the neurons survival after a glutamatergic insult, which partially mimics the processes which develop in vivo after acute cerebral ischemia. This neuronal protection develops after administering fractalkine to cultured neurons, in the model of acute ischemia, at the glutamatergic insult or even 8 hours after this insult. Findings obtained in the *in vitro* model, permitted us to propose a fractalkine neuroptrotective activity or, less probably, an endogenous mechanism neuroprotective against neuronal death following a massive release of glutamate after ischemia. However, there was no direct or indirect evidence suggesting that the *in vitro* effects of fractalkine (F) could be extended also *in vivo.* Instead, many facts did make not obvious the extention of these conclusions from the *in vitro* to the *in vivo* conditions. First, in the *in vitro* model experiments are carried out in mixed cultures with neurons and glial cells, which develop synaptic connections necessarily far from the physiological conditions. *In vivo* conditions, instead, the synaptic network is clearly different. This morphological differences may lead to different sensitivity and responsivity to the exogenous fractalkine used as a drug. Furthermore, since fractalkine in its soluble form, is known to exert a chemiotactic activity on many types of immune system cells (Vitale et al., J Immunology, 2004, 172:585-592), its intraventricular injection was destined to cause cell infiltration into the brain with side effects so strong such to discourage an *in vivo* fractalkine administration.

To date acute ischemia, for instance after cardiac arrest, did not find effective pharmacological treatments which must be done within a few hours after the ischemic insult. Presently, treatments effective against acute ischemia to interrupt toxic ischemic processes yielding to neuronal death do not exist. Looking for these treatments was a problem felt in the state of the art. The invention consists in identifying fractalkine as an useful drug to block the processes underlying acute ischemia leading to neuronal toxicity and neuronal death; and in proposing such drug as pharmacological treatment effective within the firs hours after ischemic episode. Noteworthy, ischemia represents the third cause of death, and the first cause of permanent invalidity in the most industrialized countries. Ischemia results from many pathophysiological interconnected events: the interruption of blood flux causes reduction of glucose and oxygen supply to tissues; in turn, this causes acidosis together with release of toxic substances including glutamate, with consequent increase of huge intracelluar calcium which is invariably neurotoxic. Experiments in animals and clinical findings indicate that time interval to produce irreversible damages after ischemia is about 6 hours.

The state of the art, represented by the work on the effects of fractalkine on neuron in culture, did not suggest the possibility of an in vivo application, just for the difference of neuron/glial cell synaptic connection between the in vitro system and that in vivo. Another reason was that there were physiological chemiotactic effects of fractalkine which represented a possible sideffect of fractalkine itself.

It is therefore an object of the present invention fractalkine for use as a neuroprotective agent to block, and/or to counteract, and/or to prevent neuronal toxic processes by glutamate, in particular due to cerebral ischemia, spinal lesions and/or where local blood flow of nerve tissue is interrupted in neurosurgery. A further object of the present invention is the use of fractalkine to produce a neuroprotective drug to block and/or to counteract and/or to prevent neuronal toxic processes by glutamate, specifically due to cerebral ischemia, spinal lesions and/or in case where local blood flow of nerve tissue is interrupted in neurosurgery.

### List of drawings

3 figures are enclosed with the present description showing:
figure 1, dorsal hippocampal sections upon global and transient ischemic episode and treatment with saline solution;
figure 2, a densitometric analysis of hippocampal CA1 region on animal treated with control and fractalkine;

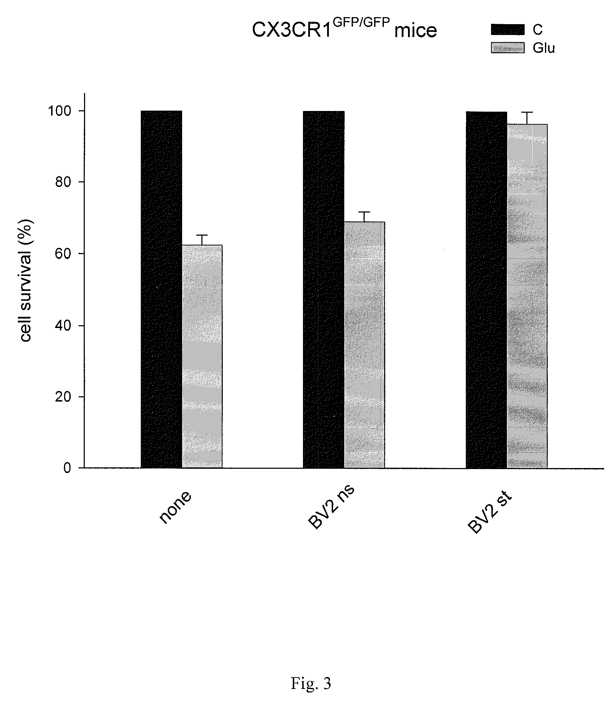

figure 3, an histogram of neuronal survival induced by conditioned medium by microglial cells stimulated by fractalkine.

In this regard, the inventors made experiments on *in vivo* animals using the model of acute ischemia following cardiac arrest in the gerbil, consisting in the transient blockage of the carotid circulation and in observing, after 3 days, the hippocampal damage following the method recently published (Cappuccio et al., Induction of Dickkopf-1, a negative modulator of the Wnt pathway, is required for the development of ischemic neuronal death, J neuroscience, 2005, March 9; 25(10): 2647-57). This study provided for information that fractalkine administration by intracerebroventricular injection (i,c,v,; 20 pmo/2L) protect pyramidal neurons in hippocampal CA1 area against cell death caused by global and transient ischemic episodes (provoked by bilateral blockage of the carotids for (6 minutes). I.c.v. infusion has been made by intracerebroventricular implant of a fixed cannula a day before the carotid occlusion and using the following stereotaxic parameters: AP -1mm; ML +1mm; DV -3mm. Animal were sacrificed after 3 days and the brain tissues were immediately frozen and kept at -80 °C. Serial coronal sections, 20 micron thickness, were processed to histological analysis of neuronal death, through Nissl staining. Results evidenced that hippocampal neuronal death was reduced from about 80% to about 40% of the control in the animals in which the transient carotid occlusion was carried on with contemporary fractalkine administration. Fig.s 1 & 2 here enclosed, show the results obtained. These findings were also obtained with fractalkine administration 1 hours after carotid occlusion.

Inventors discovered also that fractalkine (and this also forms object of the present invention), which is produced and present in the CNS, not only strongly increases neuronal survival and represents a drug for patients with acute cerebral ischemia, cranial trauma, spinal trauma, but also may be used to prevent ischemic trauma during neurosurgical operations in brain when the blood flow of specific brain zones is blocked to achieve an optimal outcome of the surgical intervention.

The animal model of rodent gerbil with transient carotid occlusion used by the inventors is considered an appropriate model for acute ischemia useful for discovering molecular mechanisms and useful drugs for patients. To date neuroprotective drugs against ischemic insult with similar efficacy as fractalkine has shown to be in animal are not available on the market.

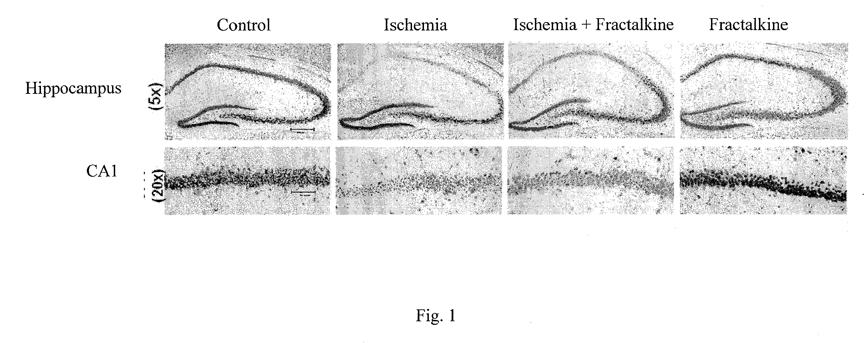

Fig. 1 illustrates sections of dorsal hippocampus from gerbils sacrificed 3 days after transient global episodes obtained with 6 min bilateral carotid occlusions, and colored by Nissl method. Just before the surgical operation of animals (both controls and carotid occluded animals) saline solution alone or containing fractalkine i.c.v. (fractalkine concentration 20 pmol/2L i.c.v.) was administered.

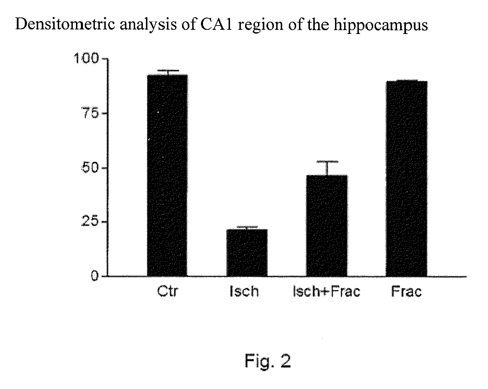

Fig. 2 illustrates quantification of findings reported in Fig.1. Specifically Fig. 2 illustrates the statistical result of the densitometric analysis of hippocampal CA1 sections under different experimental conditions. Since the viability index is represented by cell staining intensity, the finding illustrates that the treatment with fractalkine is able to increase cell viability upon ischemic insult.

### Experimental Methods.

### (A) In vitro studies: experiments of excitotoxicity by glutamate in hippocampal neurones cultured from mice CX3CR1^{-/-}

It has been investigated whether fractalkine can exert its neuroprotective action indirectly through microglia. This working hypothesis derives from the fact that in the literature contrasting data exist on the localization of the fractalkine receptors (FR) in cellular types of the nervous system. Specifically, it has been stated by some authors that fractalkine is present only on microglial cells and astrocytes (only in case of neuron inflammation); while in its membrane anchored form is expressed only in neurons. Thus, we used an immortalized murine cell line of microglia (BV2) from engeneerized mice so that they express a fluorescent protein (GFP) on the place of fractalkine, called mice CX3CR1^{GFP/GFP} (CX3CR1^{-/-}), both commercially available. With these mice cultures of hippocampal neurons have been obtained used for experiments of glutamate excitotoxicity. Findings obtained show that the conditioned medium, recovered from 18 hours after BV2 exposute to fractalkine, protects hippocampal neurons treated in culture with glutamate (fractalkine ig. 3). This effect is attributed to fractalkine but is an indirect effect on the neurons and mediated through cells of microglia stimulated by fractalkine: neuronal cultures used do not express fractalkine receptor CX₃CR1.

In experiments above reported, the growth medium is removed and kept for subsequent incubation, cells are rinsed and stimulated with glutamate (100 microM) for 30 min in Locke's buffer without MgCl₂ and additioned with glycine (10 microM) to stimulate all glutamatergic receptors. Successively, such buffer is removed, cells are rinsed and again incubated in the conditioned medium. In such a standard experiment, the neuroprotective properties of the conditioned medium of microglia stimulated with fractalkine were tested. To do that, the day before the excitotoxic insult on hippocampal neuron cultures, cultured microglial cells (BV2) are treated for 30 min with 100 nM MgCl₂: the growth medium (DMEM with 10% serum) is removed, cells are rinsed and stimulated with fractalkine (100 nM) in Locke's buffer without magnesium and adding glycine (10 nM) for 30 min; successively buffer is removed, cells are rinsed and maintained over night in Neurobasal with B27 complement and kept in the incubator (37 °C; 5% CO2). Such a medium is then added in 1:1 ratio to conditioned medium of hippocampal cultures, and after 20 hours the neuronal survival is analysed.

### (B) In vivo studies: experiments of permanent ischemia in rat

These experiments were meant to be a continuation of the in vivo experiments carried out on gerbil, using a model of permanent ischemia in rat. Preliminary findings show that ischemic rats treated with fractalkine show a rescue of sensory motor functions. In contrast, control rats do not exhibit such a rescue (as supported by high scores obtained in De Ryck test and by low scores obtained through the Foot faul test). Finally, the ischemic volume in rat treated with fractalkine is significantly reduced. In experiments above reported, rats (Sprague-Dawley strain) are kept in a room at temperature and light controlled (12 hr light/dark cycle). In the ischemia experiments, male rats were used (7-9 week-old; 250-300 g weight). Permanent ischemia has been obtained through a first occlusion of right carotid, then the medial cerebral artery is cauterized, and finally the controlateral common carotid criteria is occluded for 1 h. Fractalkine has been administered intracerebroventricularly (10 pmol/2L i.c.v.) at the same time of the ischemic insult.

24 hours after ischemia induction, rats undergo 3 different tests permitting to estimate the sensory motor functions (Bederson's; De Rick's and Foot-fault test) to verify whether neurological deficits have arisen or not. Specifically, a low score in Bederson's and De Ryck tests suggests a relevant ischemic damage; on the contrary, a low score in Foot-fault test shows a less severe ischemic damage. In order to evaluate the damage extension at the tissue level, rats are sacrificed 24 h after the ischemic insult and after the tests have been carried out, their brain removed and treated to obtain serial slices, 1 microm thickness, which are then incubated in 1% TTC (Triphenyltetrazolium chloride) and stained in 4% PFA. Extension of ischemic damage is evaluated by digital imaging analysis.

Further object of the present invention are also pharmaceutical compositions with neuroprotective action to block and/or counteract and/or prevent neurotoxic processes by glutamate, and/or in case of blockage of local irroration of the nerve tissue in a neurosurgery, comprising fractalkine and, or eccipient pharmaceutically tolerable. In said compositions fractalkine concentration is comprised between 1-40 pM/2L.

## Claims

1. Fractalkine for use as a neuroprotective agent to block and/or counteract and/or prevent neurotoxic processes induced by glutamate and/or in case of blockage of blood circulation in localized brain regions in neurosurgery.

2. Fractalkine according to claim 1, in which said neurotoxic processes are due to cerebral ischemia or spinal cord lesions.

3. Fractalkine according to claims 1 or 2 administered at concentrations in the range between 1 and 40 pmol/2L.

4. Use of fractalkine for the manufacture of a neuroprotective drug to block and/or counteract and/or prevent neurotoxic processes induced by glutamate and/or in case of blockage of blood circulation in localized brain regions in neurosurgery.

5. The use according to claim 4, wherein said neurotoxic processes are consequent to cerebral ischemia, or spinal cord lesions.

6. The use according claims 4 or 5, wherein fractalkine is administered at concentrations in the range between 1 and 40 pmol/2L.

7. Pharmaceutical compositions with neuroprotective action to block and/or counteract and/or prevent neurotoxic processes induced by glutamate and/or in case of blockage of blood circulation in localized brain regions in neurosurgery comprising fractalkine and additives and/or excipients pharmacologically tolerable.

8. Pharmaceutical compositions according to claim 7, wherein said processes of neurotoxicity are due to cerebral ischemia and/or spinal cord lesions.

9. Compositions according to claims 7 and/or 8, wherein fractalkine is present at concentrations in the range between 1 and 40 pmol/2L.
